# EUROPEAN PATENT APPLICATION

(11) **EP 3 335 627 A1**
(43) Date of publication of application: **20.06.2018**
(21) Application number: 17207252.2
(22) Date of filing: 14.12.2017
(51) Int. Cl.: A61B 5/00

(54) **PHOTOACOUSTIC CATHETER AND PHOTOACOUSTIC CATHETER SYSTEM**

(30) Priority: 16.12.2016 JP 2016243983
(71) Applicant: Hitachi, Ltd., Chiyoda-ku Tokyo 100-8280 (JP)
(72) Inventor: TANAKA, Tomohiko, Chiyoda-ku, Tokyo 100-8280 (JP); IMAI, Ryo, Chiyoda-ku, Tokyo 100-8280 (JP); TAKEZAKI, Taiichi, 100-8280, Tokyo (JP); IKEDA, Teiichiro, Chiyoda-ku, Tokyo 100-8280 (JP); NAKAMURA, Toshiteru, Chiyoda-ku, Tokyo 100-8280 (JP); MATSUDA, Takahiro, Chiyoda-ku, Tokyo 100-8280 (JP); ONOE, Shinsuke, Chiyoda-ku, Tokyo 100-8280 (JP)
(74) Representative: Addiss, John William

(57) **Abstract**

It is an object to detect a sound wave without changing a direction of an acoustic element even if light is radiated in any direction. This object is achieved by means of a driving device 3 that drives so as to radiate light in a predetermined direction to an advancing direction of the catheter 101 and an acoustic element 1 that receives a sound wave generated by the radiated light, in which the acoustic element 1 is provided in a circumference direction of the catheter 101 and provided in a direction having a predetermined angle to an advancing direction of the light. Furthermore, a plurality of acoustic elements 1 are arranged in an array to form an acoustic element array 10.

## Description

### Technical Field

The present invention relates to a technology of a photoacoustic catheter and a photoacoustic catheter system using a photoacoustic technology.

### Background Art

Since a treatment using a catheter (catheter treatment) is a surgical operation with fewer burdens on patients as compared with open chest surgery, the treatment is increasingly used. In such a catheter treatment, a radiographic visualization is used to visualize blood vessels and the like in a body for specifying a stenosed portion, placing a guide wire, and performing a postoperative evaluation. However, the radiographic visualization is not preferable for patients and doctors. Therefore, a visualization technology for reducing the radiographic visualization is required.

Such a technology is a three-dimensional intravascular imaging technology (catheter imaging method) using a catheter. In this technology, a three-dimensional image can be acquired by rotating the catheter around an axis while moving the catheter and performing imaging by a catheter tip end portion.

A three-dimensional structure that is intuitively excellent can be provided to a doctor by such a catheter imaging method. Furthermore, a catheter placing operation can be supported by such a technology. Here, in the current catheter imaging method, a motor of about 1800 rpm is used for rotation around the catheter axis. Here, the motor is configured to rotate the catheter itself in a direction perpendicular to the catheter axis. That is, the motor rotates the catheter itself in a twisting direction.

The rotational speed can obtain a sufficient frame rate at a time of planar imaging. However, it is insufficient for real-time three-dimensional imaging in stereoscopic imaging. That is, in the stereoscopic imaging in which a lot of images are acquired as compared with planar imaging, it takes time for imaging at the above-described rotational speed.

In addition, as the catheter imaging method for visualizing inside a blood vessel using light or a sound wave, there are optical coherence tomography (OCT) imaging, intravascular ultrasound (IVUS) imaging, intravascular photoacoustic (IVPA) imaging, and the like. These three methods have a separate object for three-dimensional imaging.

PTL 1 discloses a catheter type photoacoustic probe that "an outer sheath 31 is inserted into a lumen. An inside portion of the outer sheath 31 is filled with a liquid and an inner sheath 32 is movable within the outer sheath 31 along the lumen in an extending direction. An ultrasonic detector 21 and a light irradiating portion composed of, for example, a lens 22 and a mirror 23 are arranged inside the inner sheath 32. The outer sheath 31 has filters 24 and 25 for filtering the liquid present outside the outer sheath." (refer to Abstract).

### Citation List

### Patent Literature

PTL 1: JP-A-2013-22171

### Summary of Invention

### Technical Problem

As a technology for increasing a rotational speed of the catheter in the catheter imaging method, there is an optical fiber scanning technology. The optical fiber scanning technology is a technology for controlling a direction (imaging direction) of light emitted from a tip end of an optical fiber by controlling the tip end of the optical fiber. According to this, the direction of the light emitted from the tip end of the optical fiber can be changed without rotating the catheter itself.

In a case where a transmitting and receiving of a signal is performed with light, real-time three-dimensional imaging can be performed with the optical fiber technology alone. However, in the catheter type photoacoustic probe (catheter) as described in PTL 1, a sound wave generated by light radiated on an object to be measured (living body) is detected by the acoustic element. Only one acoustic element is provided in the catheter described in PTL 1. Therefore, the catheter type photoacoustic probe (catheter) as described in PTL 1 may tilt the acoustic element in the direction of radiating the light. That is, in the fiber scanning technology, even if the direction of the light to be emitted from the tip end of the optical fiber is changed without rotating the catheter itself, since only one acoustic element is provided, it is necessary to direct the acoustic element in a direction of flight of the sound wave generated by the radiated light. That is, resultantly, it is required to rotate the catheter itself.

As described above, the photoacoustic technology described in PTL 1 is not compatible with the optical fiber scanning technology.

That is, if the fiber scanning technology and the technique of changing the receiving direction of the acoustic element which has a size larger than that of the aperture of the optical fiber by 10-fold are used, the diameter itself of the catheter becomes a diameter of several millimeters. Such a diameter becomes a major disadvantage in actual clinical practice.

That is, in the photoacoustic technology, the acoustic element is directed in the direction of light radiation. However, when the direction of the acoustic element is changed using the optical fiber scanning technology, a mechanism for changing the direction of the acoustic element is required in addition to the large size of the acoustic element itself. In this regard, the diameter of the catheter itself becomes greater.

The invention has been made in view of such a background, and an object of the invention is to detect the sound wave without changing the direction of the acoustic element even when the light is radiated in any direction.

### Solution to Problem

In order to solve the above-described problems, the invention includes a driving unit that drives so as to radiate light in a predetermined direction to an advancing direction of the catheter and an acoustic detection unit that receives a sound wave generated by the radiated light, in which the acoustic detection unit is provided in a circumference direction of the catheter and provided in a direction having a predetermined angle to an advancing direction of the light.

The other solutions will be described in embodiments. Advantageous Effects of Invention

According to the invention, the sound wave can be detected without changing the direction of the acoustic element even when the light is radiated in any direction.

### Brief Description of Drawings

[Fig. 1] Fig. 1 is a diagram illustrating a configuration of a tip end portion of a catheter to be used in a first embodiment.
[Fig. 2] Fig. 2 is a diagram illustrating a light radiation mechanism in the tip end portion of the catheter.
[Fig. 3] Fig. 3 is a functional block diagram illustrating a configuration of a photoacoustic catheter system to be used in the first embodiment.
[Fig. 4] Fig. 4 is a functional block diagram illustrating a hardware configuration example of an image generation apparatus to be used in the first embodiment.
[Fig. 5] Fig. 5 is a flowchart illustrating a processing procedure in the photoacoustic catheter system to be used in the first embodiment.
[Fig. 6] Fig. 6 is a diagram illustrating a group of acoustic element arrays to be used in a second embodiment.
[Fig. 7] Fig. 7 is a diagram (Part 1) illustrating a pattern example of grouping of the acoustic elements.
[Fig. 8] Fig. 8 is a diagram (Part 2) illustrating a pattern example of grouping of the acoustic elements.
[Fig. 9] Fig. 9 is a functional block diagram illustrating a hardware configuration example of an image generation apparatus to be used in the second embodiment.
[Fig. 10] Fig. 10 is a flowchart illustrating a processing procedure in the photoacoustic catheter system to be used in the second embodiment.
[Fig. 11] Fig. 11 is a diagram illustrating a group of acoustic element arrays to be used in a third embodiment.
[Fig. 12] Fig. 12 is a diagram illustrating an input sound wave waveform and a signal waveform in each group.
[Fig. 13] Fig. 13 is a flowchart illustrating a processing procedure in the photoacoustic catheter system to be used in the third embodiment.
[Fig. 14] Fig. 14 is a diagram illustrating a simulation condition.
[Fig. 15] Fig. 15 is a diagram illustrating a sound wave waveform output from a sound source.
[Fig. 16] Fig. 16 is a diagram illustrating a waveform of a detection signal in acoustic elements 1A to 1D.
[Fig. 17] Fig. 17 illustrates a simulation result of the group shown in the second embodiment.
[Fig. 18] Fig. 18 illustrates a simulation result of the group shown in the third embodiment.
[Fig. 19] Fig. 19 is a diagram illustrating a result of Delay & Sum in a method used in the second embodiment and the method used in the third embodiment.
[Fig. 20] Fig. 20 is a diagram for illustrating a configuration of a tip end portion of a catheter to be used in a fourth embodiment.
[Fig. 21] Fig. 21 is a diagram for illustrating another configuration example of the tip end portion of the catheter to be used in the fourth embodiment.
[Fig. 22] Fig. 22 is a diagram for illustrating a configuration of a tip end portion of a catheter to be used in a fifth embodiment.

### Description of Embodiments

Hereinafter, a detailed description will be given of embodiments of the invention (referred to as "Embodiment") with reference made to the drawings where appropriate. The invention relates to a photoacoustic technique of a catheter. The catheter is a tube having a diameter of about 0.5 to 3 mm to be used for medical use (mainly observation inside the blood vessel).

### [First Embodiment]

### (Catheter Tip End Portion)

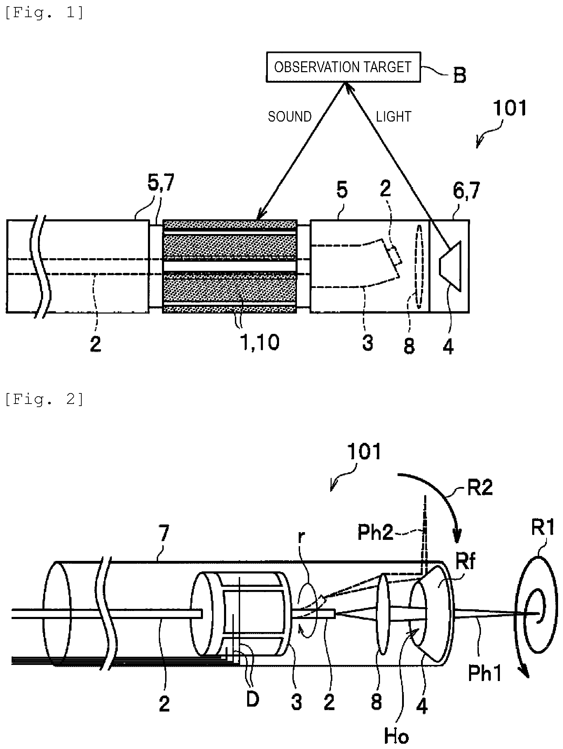

Fig. 1 is a diagram illustrating a configuration of a tip end portion of a catheter to be used in a first embodiment. Fig. 2 is a diagram illustrating a light radiation mechanism in the tip end portion of the catheter.

A photoacoustic catheter 101 (hereinafter, simply referred to as a catheter 101; catheter portion) includes array-like acoustic elements (acoustic detection unit) 1 around the catheter 101 itself (about the axis of the catheter 101). That is, the acoustic elements 1 are arranged in an array to form an acoustic element array 10. When the light emitted from an optical fiber (optical fiber portion) 2 provided inside the catheter 101 is radiated to an observation target B that is a living body, the observation target B emits heat, and as a result, the volume of the observation target B expands. As a result of this volume expansion, the acoustic element 1 detects the generated sound wave. That is, the acoustic element 1 receives the sound wave generated by the radiated light.

In Fig. 1, the acoustic element 1 is indicated by dots.

First, the light radiation mechanism in the catheter 101 to be used in the first embodiment will be described below.

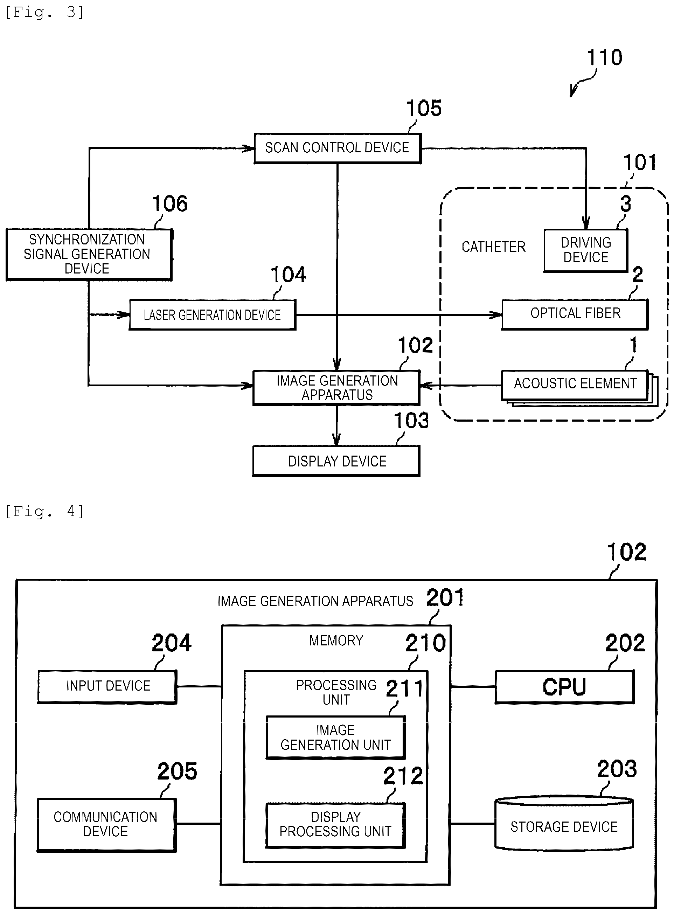

In the catheter 101, the signal light is transmitted from a laser generation device (light source unit) 104 (refer to Fig. 3) by an optical fiber 2. As illustrated in Fig. 2, it is preferable that in a driving device (driving unit) 3, a piezoelectric element such as a cylindrical 4-pole PZT element (hereinafter, simply referred to as a PZT element) is used, for example. As illustrated in Fig. 2, by applying a potential difference between facing electrodes in the PZT element via a conductor wire D, the PZT element is bent in that direction. A voltage to be applied to two pairs of facing electrodes is defined as a sine wave, and by shifting the phase by π/2, the tip end of optical fiber 2 passed through the PZT element can be rotated as indicated by a reference symbol r in Fig. 2. A rotation radius of the optical fiber 2 (the radius of rotation indicated by the reference symbol r in Fig. 2) is controlled by an amplitude of a sinusoidal voltage to be applied to the PZT element.

In this manner, the driving device 3 is driven so as to radiate light in a predetermined direction to an advancing direction of the catheter 101.

In addition, as shown in Figs. 1 and 2, the catheter 101 includes a reflecting mirror (mirror portion) 4 on the tip end side of the catheter 101 to the driving device 3. The reflecting mirror 4 is provided in the vicinity of a light emitting end (hereinafter, referred to as a tip end) of the optical fiber 2. The reflecting mirror 4 has a hollow (having a hollow portion Ho in Fig. 2) truncated cone shape. A reflection portion Rf illustrated in Fig. 2 is formed by coating around the reflecting mirror 4 with a metal or the like.

In Fig. 1, the reflection portion Rf is formed on the entire side of the side surface of the reflecting mirror 4. However, it is not limited thereto. The reflection portion Rf may be formed only on the portion irradiated with the light emitted from the tip end of the optical fiber 2 or the reflection portion Rf may not be formed on a part of the side surface of the reflecting mirror 4. By setting the shape of the reflecting mirror 4 to a truncated cone shape, the reflecting mirror 4 which can reflect the light emitted from the tip end of the optical fiber 2 to the side can be easily produced. In addition, since the reflecting mirror 4 has the hollow portion Ho, the reflecting mirror 4 that allows the light emitted from the tip end of a fiber 11 to transmit (pass through) a center portion and travel straight can be easily produced.

The light emitted from the tip end of the optical fiber 2 diverges at a unique angle to the optical fiber 2. Therefore, as shown in Fig. 1, a lens 8 for condensing the light to the observation target B is provided. That is, the lens 8 is a convex lens having a refractive index and a radius of curvature such that when the optical fiber 2 is bent so as to face the reflection portion Rf of the reflecting mirror 4, the lens 8 bends the light so that the light proceeds in the direction of the reflection portion Rf of the reflecting mirror 4 and the light reflected by the reflecting mirror 4 is substantially condensed on the observation target B. By providing such a lens 8, the light can be bent so that the light proceeds in the direction of the reflecting mirror 4, and the light is bent so as to substantially condense the light on the observation object B (so as to condense at least in a vicinity of the observation target B). A reflecting mirror 4 for switching the observation between the front side and the side thereof is provided on the tip end side of the catheter 101 with respect to the lens 8.

In addition, in a case where the light proceeds to the front side through the hollow portion Ho of the reflecting mirror 4, the light is condensed on the observation target B by the lens 8.

As illustrated in Fig. 1, these elements are covered with a cover 5 (7) having mechanical characteristics of the catheter 101. However, the cover of the portion that transmits the light is formed of a transparent resin (a transparent cover 6 (7)) or the like.

### (Operation Outline)

Hereinafter, an operation outline of the light radiation mechanism at the tip end portion of catheter 101 will be shown mainly with reference to Fig. 2.

In Fig. 2, among the configuration of the tip end portion of the catheter 101, only the minimum configuration necessary for explaining the operation is shown. Specifically, the acoustic element array 10 is omitted, and the cover 5 and the transparent cover 6 are omitted to set a cover 7. Furthermore, in Fig. 2, the driving device 3 is shown as a simplified diagram.

In a case where the side of the catheter 101 is observed, the driving device 3 bends the optical fiber 2 largely with the driving device 3 (the PZT element) as indicated by a thick broken line. Here, the "bends largely" means that the light emitted from the tip end of the optical fiber 2 is bent at an angle such that the light is radiated on the side surface (the reflection portion Rf) of the reflecting mirror 4 through the lens 8. As indicated by a thin broken line Ph2, the light emitted from the optical fiber 2 in this state becomes parallel light by the lens 8 and the light is reflected by the reflection portion Rf provided on the side surface of the reflecting mirror 4 to be radiated by the catheter 101.

A thick broken line in Fig. 2 indicates the optical fiber 2 bent by the driving device 3 as described above.

On the other hand, in a case where the front of the catheter 101 is observed, the driving device 3 bends the optical fiber 2 slightly or does not bend the optical fiber 2 at all. Here, "bends slightly (does not bend)" means that the light emitted from the tip end of the optical fiber 2 is bent through the lens 8 so as to pass through the inside of the hollow portion Ho of the reflecting mirror 4. The light emitted from the optical fiber 2 in this state is condensed by the lens 8 as indicated by the symbol Ph1, passes through the hollow portion Ho of the reflecting mirror 4, and is radiated to the front side of the catheter 101.

That is, in a case where the tip end of the optical fiber 2 rotates as indicated by a symbol r, and in a case where the rotation radius (curvature) thereof is equal to or more than a certain value, the light emitted from the tip end of the optical fiber 2 is reflected by the reflection portion Rf of the reflecting mirror 4 (symbol Ph2). As a result, the periphery of the catheter 101 is observed in a circular shape (symbol R2).

On the other hand, when the rotational radius (curvature) of the tip end of the optical fiber 2 is less than a certain value, the light emitted from the tip end of the optical fiber 2 passes through the hollow portion Ho of the reflecting mirror 4 and is radiated to the front side (symbol Ph1).

In this manner, in a case where the light emitted from the tip end of the optical fiber 2 is not reflected by the reflection portion Rf of the reflecting mirror 4, the emitted light is radiated to the front side of the catheter 101. In addition, in a case where the light emitted from the tip end of the optical fiber 2 is reflected by the reflection portion Rf of the reflecting mirror 4, the emitted light is radiated to the side of the catheter 101. In this manner, it is possible to switch between the observations on the front side and the side without replacing the catheter 101.

As illustrated in the symbol R1, in a case where the light is radiated to the front side, it is preferable that the radius of rotation of the tip end of the optical fiber 2 changes such that the light is radiated spirally to the observation object B (refer to Fig. 1).

As illustrated in Fig. 2, controlling the radiation direction of light by rotating the tip end of the optical fiber 2 is referred to as optical fiber scan control, or simply as a scan.

Fig. 1 is explained again.

As described above, the acoustic elements 1 are provided in an array around the catheter 101 (the acoustic element array 10). The number of acoustic elements 1 is about 1 to 1200 for one catheter 101 (the acoustic element array 10). In a case where the number of the acoustic elements 1 is one, the acoustic element 1 having a tubular shape is provided around the catheter 101. The configuration in Fig. 1 is a configuration for detecting the sound wave generated on the side of the catheter 101. In this manner, the acoustic element 1 is provided in the circumference direction of the catheter 101 and is provided in a direction having a predetermined angle with respect to an advancing direction of the light.

By providing a plurality of arrayed acoustic elements 1 on the side surface of the catheter 101 as described above, the sound waves can be received over the entire side surface of the catheter 101. Accordingly, there is no need to direct the acoustic element 1 in the light radiation direction (direction of flight of sound waves), and reducing the size of the catheter 101 used for photoacoustic technology can be obtained.

### (System)

Fig. 3 is a functional block diagram illustrating a configuration of a photoacoustic catheter system to be used in the first embodiment.

A photoacoustic catheter system (catheter system) 110 includes the catheter 101, an image generation apparatus 102, a display device 103, a laser generation device 104, a scan control device 105, and a synchronization signal generation device 106.

Since the catheter 101 and the acoustic element 1, the optical fiber 2, and the driving device 3 configuring the catheter 101 are already described with reference to Figs. 1 and 2, the description here is omitted.

The image generation apparatus 102 reconstructs an image based on a detection signal acquired from the acoustic element 1 in the catheter 101 and displays the reconstructed image on the display device 103.

The laser generation device 104 sends a laser and introduces the laser transmitted through the optical fiber 2. As the laser to be sent, a pulse laser is preferable.

The scan control device 105 is configured to control a scan operation of the optical fiber 2 described in Fig. 2.

The synchronization signal generation device 106 synchronizes the laser generation device 104 and the scan control device 105 and sends the synchronized signal to the image generation apparatus 102. The image generation apparatus 102 can manage which laser is sent in what state of the scan of the optical fiber 2 by the synchronized signal. Furthermore, the acoustic element 1 can manage in which direction the laser is emitted, the detection signal is acquired. The synchronization signal generation device 106 is often incorporated in the laser generation device 104.

The scan control device 105 sends a driving signal to the driving device 3 according to the synchronized signal to drive the driving device 3. Furthermore, the scan control device 105 sends the driving signal sent to the driving device 3 to the image generation apparatus 102.

### (Image Generation Apparatus)

Fig. 4 is a functional block diagram illustrating a hardware configuration example of an image generation apparatus to be used in the first embodiment. Fig. 3 is referred to when necessary.

The image generation apparatus 102 includes a memory 201, a central processing unit (CPU) 202, a storage device 203 such as a hard disk (HD), an input device 204, and a communication device 205.

The input device 204 is a keyboard, a mouse, or the like. The communication device 205 receives the detection signal from the acoustic element 1 and receives a synchronized signal sent from the synchronization signal generation device 106.

The memory 201 is a random access memory (RAM) or the like. A program stored in the storage device 203 is loaded into the memory 201 and the CPU 202 executes the loaded program to specify a processing unit 210, and an image generation unit 211 and a display processing unit 212 configuring the processing unit 210.

The image generation unit 211 reconstructs an image based on the detection signal received from the acoustic element 1 and the synchronized signal received from the synchronization signal generation device 106.

The display processing unit 212 displays the reconstructed image on the display device 103.

### (Flowchart)

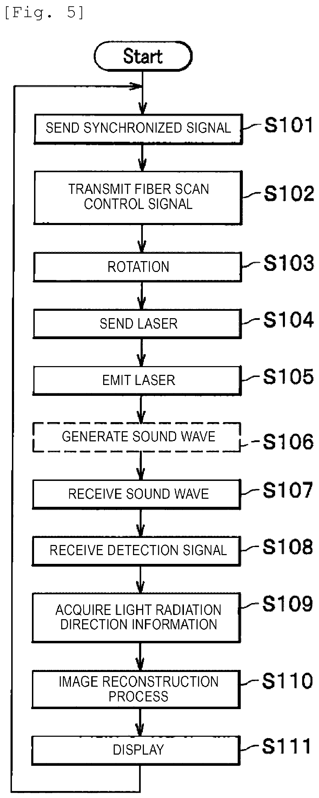

Fig. 5 is a flowchart illustrating a processing procedure in the photoacoustic catheter system to be used in the first embodiment.

First, the synchronization signal generation device 106 sends the synchronized signal (S101). Each of the image generation unit 211 of the laser generation device 104, the scan control device 105, and the image generation apparatus 102 receives the synchronized signal.

The scan control device 105 transmits an optical fiber scan control signal to the driving device 3 (S102). Specifically, the optical fiber scan control signal is a sinusoidal voltage to be transmitted to the electrode of the PZT element of the driving device 3.

The driving device 3 rotates with the amplitude and the phase according to the received signal (S103). The optical fiber 2 also rotates in accordance with the rotation of the driving device 3.

The laser generation device 104 generates a laser and sends the generated laser to the optical fiber 2 (S104).

When the laser reaches the tip end of the optical fiber 2, the laser is emitted from the tip end of the optical fiber 2 (S105). The emitted laser is reflected by the reflection portion of the reflecting mirror 4 and is radiated on the observation target (living body).

An observation target irradiated with the laser generates thermal expansion, and the observation target generates a sound wave by expansion thereof (S106).

The acoustic element 1 receives the generated sound wave (S107). The acoustic element 1 transmits a detection signal to the image generation apparatus 102.

The image generation unit 211 of the image generation apparatus 102 receives the detection signal from the acoustic element 1 (S108) and acquires light radiation direction information from the synchronization signal generation device 106 and the scan control device 105 (S109). The light radiation direction information includes synchronization information acquired from the synchronization signal generation device 106 and optical fiber scan control information acquired from the scan control device 105 (that is, phase information of the sinusoidal voltage to be sent to the electrode of the driving device 3).

In the image generation unit 211, the detection signal received in Step S108 and acquired in Step S109 from the synchronizing signal included in the light radiation direction information specifies whether the sinusoidal voltage sent to the electrode of the drive device 3 is derived from the emitted light at what phase. The operation of the driving device 3 can be specified and the direction of the tip end of the optical fiber 2 can also be specified by the phase of the sinusoidal voltage sent to the electrode of the driving device 3. In this manner, the image generation unit 211 specifies the radiation direction of the light (the direction of flight of the sound wave).

The image generation unit 211 of the image generation apparatus 102 performs image reconstruction process based on the detection signal received in step S108 and the light radiation direction information received in step S109 (S110).

When image reconstructing is performed, a Delay & Sum technique may be used in consideration of the distance between a sound wave generation source and each position of the acoustic elements 1.

The display processing unit 212 displays the reconstructed image in step S110 on the display device 103 (S111).

As shown in the first embodiment, since the acoustic element 1 is provided in the circumference direction of the catheter (about the axis of the optical fiber 2) and provided in a direction having a predetermined angle to the advancing direction of the light, the sound wave can be detected even when the sound wave comes flying from any direction. That is, according to the catheter 101 shown in the first embodiment, the sound waves can be detected without changing the direction of the acoustic element 1 even when the light is radiated in any direction.

Furthermore, by providing a plurality of acoustic elements 1 in the circumference direction of the catheter 101, since detection signals can be processed for each acoustic element 1, the detection resolution can be improved.

By providing the acoustic element 1 in the circumference direction of the side surface of the catheter 101, the sound waves derived from the light radiated to the side of the catheter 101 can be detected.

In addition, since the catheter 101 has the driving device 3 and the reflecting mirror 4, high-speed scanning can be performed, and it is possible to switch between the observations on the side and the front side thereof.

### [Second Embodiment]

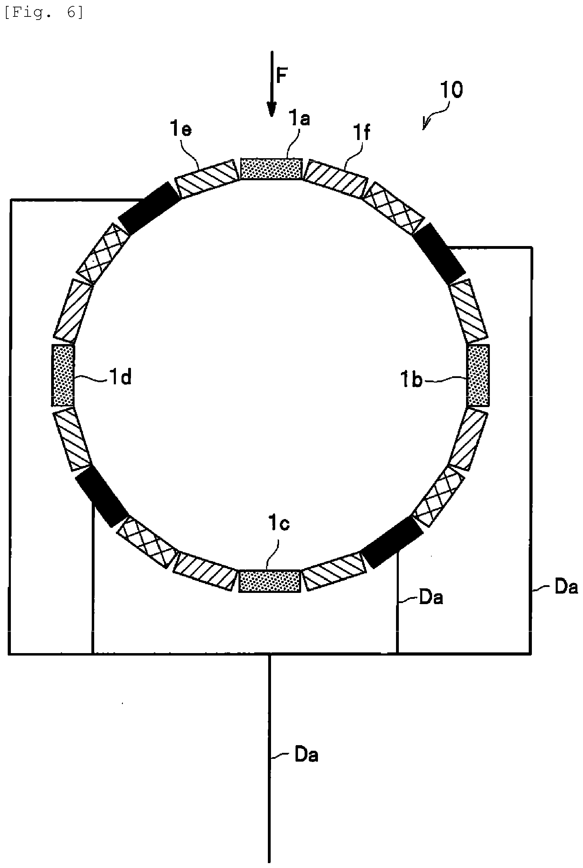

Hereinafter, a second embodiment of the invention will be described with reference to Figs. 6 to 10. In the second embodiment, since the configuration of the photoacoustic catheter system 110 is similar to the configuration illustrated in Fig. 3, the description here will be omitted. Further, in the description of the third embodiment, Figs. 1 to 3 are referred to when necessary.

In the second embodiment, the acoustic elements 1 are grouped.

### (Acoustic Element Array)

Fig. 6 is a diagram illustrating a group of acoustic element arrays to be used in the second embodiment.

Fig. 6 is a schematic diagram of the acoustic element array 10 including twenty acoustic elements 1 as viewed from the axial direction of the catheter 101 (refer to Fig. 1).

In Fig. 6, the acoustic elements 1 indicated by the same hatching are grouped by the same wire. In Fig. 6, only a wire Da of the group of the acoustic elements 1 painted with black is illustrated. Similarly, the wires of the group of the acoustic elements 1 subjected to the other hatching are common.

Accordingly, in the second embodiment, the acoustic elements 1 illustrated by the same hatching in Fig. 6 are grouped, and the wires of the acoustic elements 1 belonging to the same group are common. In Fig. 6, twenty acoustic elements 1 are divided into five groups.

In this manner, the number of wires can be reduced and the diameter of the catheter 101 can be reduced.

At this time, grouping is performed according to the following rules.

The acoustic elements 1 are arranged so as not to be adjacent to each other at least. Specifically, when the number of acoustic elements 1 is defined as N and the number of groups is defined as n, the acoustic element 1 of the same group is present at every (N/n)-1.

More specifically, the acoustic elements 1, which are directed in a direction close to the direction (the light radiation direction) of flight of the sound wave, and the acoustic elements 1 which face in different directions are grouped. Specifically, in Fig. 6, acoustic elements 1a to 1d that are given the same hatching belong to the same group. In a case where a sound wave F arrives from the direction indicated by the solid arrow in Fig. 6, the acoustic element 1a strongly detects the sound wave F. However, since the acoustic element 1 shown in the other acoustic elements 1b to 1d shifts from the direction in which the sound wave F comes, the signal intensity is low. Accordingly, even if the acoustic elements 1a to 1d are grouped and the wire is commonly used, if the direction of the sound wave F is known, it is possible to specify the acoustic element 1 detecting the sound waves F. The direction of the sound wave F can be found more easily by the management of the optical fiber scan control.

In addition, an acoustic element 1e and an acoustic element 1f have the signal intensity similar to those of the acoustic element 1a to the sound wave F. Accordingly, if the acoustic elements 1a, 1e, and 1f configure the same group and the wire is used in common, even if the direction of the sound wave F is known, the detection signals of each of the acoustic elements 1 overlap each other, and it is impossible to found that what kind of acoustic element 1 among the acoustic elements 1a, 1e, and 1f sends what kind of the detection signal.

Accordingly, the acoustic elements 1a, 1e, and 1f are not in the same group.

In this manner, by using the wires of the acoustic elements 1 with high signal intensity and the acoustic element 1 with low signal intensity in common to the sound wave F coming from a certain direction, a grouping of the acoustic elements 1 is performed.

The diameter of the catheter 101 is about 0.5 to 3 mm, the diameter of the wire is about 0.1 mm, and the diameter of the optical fiber 2 is about 0.1 to 0.2 mm.

### (Variation of Grouping)

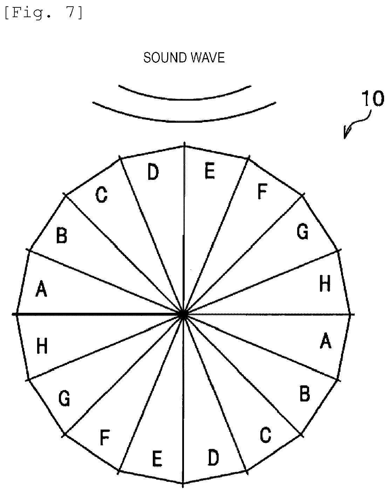

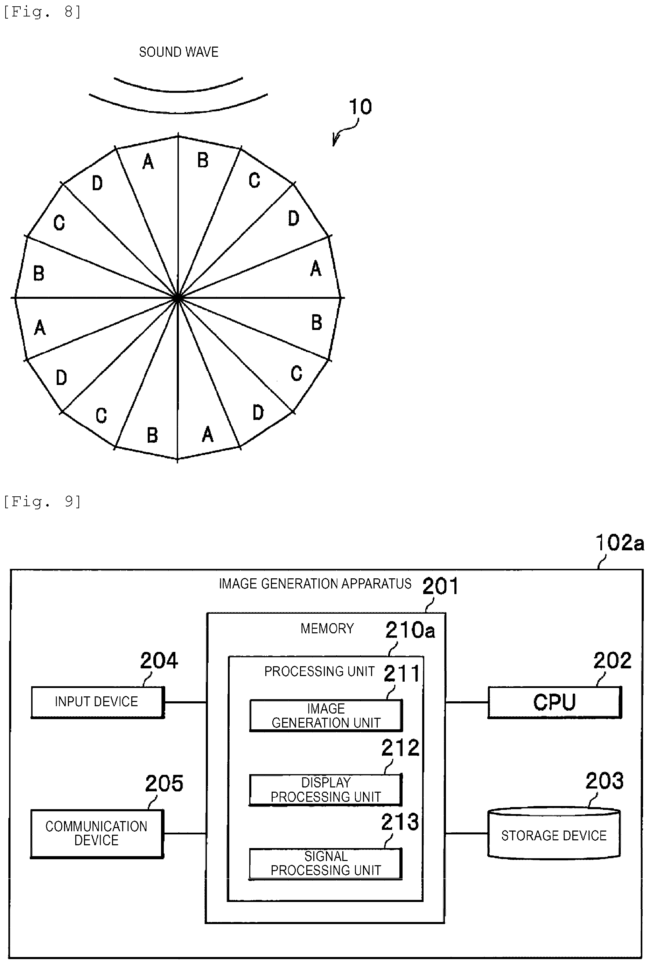

Figs. 7 and 8 are diagrams illustrating a pattern example of grouping of the acoustic elements.

Here, Figs. 7 and 8 are schematic diagrams of the acoustic element array 10 provided with the sixteen acoustic elements 1 as viewed from the axial direction of the catheter 101.

Fig. 7 illustrates sixteen acoustic elements 1 in two groups and Fig. 8 illustrates sixteen acoustic elements 1 in four groups. In Figs. 7 and 8, the acoustic elements 1 indicated by "A" belong to the same group, and the acoustic elements 1 indicated by "B" belong to the same group. The same also applies to "C" to "H".

The number N of acoustic elements 1 in one catheter 101 varies from 1 to 1200 as described above (in the example of Figs. 7 and 8, N=16). Since the acoustic element 1 with high signal intensity and the acoustic element 1 with low signal intensity are grouped in the same group, the grouping is performed on a point object as illustrated in Figs. 7 and 8.

Here, as the group pattern, a pattern of 360°/n can be considered. n is the number of groups, n = 2 to N/2 holds true (N is the number of acoustic elements 1 in the acoustic element array 10, and N=16 in the example of Figs. 7 and 8). At this time, the number of wires is N/n. For example, in the grouping illustrated in Fig. 7, the number of wires is originally sixteen and finally decreases to eight, and finally, the number of wires decreases to four in the grouping shown in Fig. 8.

For example, the following grouping can be considered.
For each 360°/2=180 degrees (Two groups): Corresponds to Fig. 7
For each 360°/3=120 degrees (Three groups)
For each 360°/4=90 degrees (Four groups): Corresponds to Fig. 8
For each 360°/5=72 degrees (Five groups)
For each 360°/6=60 degrees (Six groups)

In the second embodiment, since the wires of the acoustic elements 1 belonging to the same group is simply used in common, the signal sent to an image generation apparatus 102a (refer to Fig. 9) becomes the signal to which the signal of the acoustic element 1 belonging to the group is added. However, as described above, by grouping the acoustic element 1 directed in a direction close to the light radiation direction (the direction of flight of the sound wave) and the elements facing in different directions, the degree of interference of the signals of the acoustic elements 1 belonging to the same group is low.

### (Image Generation Apparatus)

Fig. 9 is a functional block diagram illustrating a hardware configuration example of an image generation apparatus to be used in the second embodiment.

In Fig. 9, the same reference numerals are given to the same configurations as those in Fig. 4, and a description thereof will be omitted.

The image generation apparatus 102a illustrated in Fig. 9 is different from the image generation apparatus 102 illustrated in Fig. 4 in that a signal processing unit 213 in the memory 201 is executed by the CPU 202 as a unit constituting the processing unit 210a. That is, the difference between the image generation apparatus 102 illustrated in Fig. 4 and the image generation apparatus 102a illustrated in Fig. 9 is the presence or absence of the signal processing unit 213.

The signal processing unit 213 specifies the detection signal of the main component in each group (each wire).

### (Flow Chart)

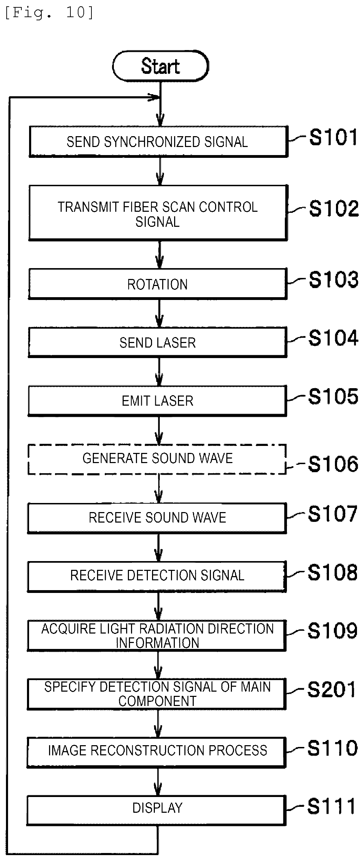

Fig. 10 is a flowchart illustrating a processing procedure in the photoacoustic catheter system to be used in the second embodiment. In Fig. 10, processes similar to those in Fig. 5 are denoted by the same step numbers, and description thereof will be omitted.

In step S108, the image generation apparatus 102a receives the detection signal and acquires the light radiation direction information from the synchronization signal generation device 106 and the scan control device 105 in step S109. Thereafter, the signal processing unit 213 of the image generation apparatus 102a specifies the detection signal of the main component in each group (each wire) (S201). The signal processing unit 213 uses the signal at the earliest time in each group or the signal with the highest signal intensity as the detection signal of the main component.

The image generation unit 211 of the image generation apparatus 102a performs image reconstruction processing based on the detection signals of the main components in each group specified in step S201 and the light radiation direction information received in step S109 (S110).

The display processing unit 212 performs the process of step S111.

According to the second embodiment, since the predetermined acoustic element 1 among the acoustic elements 1 configuring the acoustic element array 10 is grouped and the wire of the acoustic element 1 belonging to the same group is used in common, the number of the wires can be reduced. Accordingly, reducing the diameter of the catheter 101 can be obtained.

Furthermore, in the second embodiment, the acoustic element 1 directed in a direction close to the light radiation direction and the acoustic element 1 directed in different directions are grouped. That is, the plurality of acoustic elements 1 form a group, and the wires of the acoustic elements 1 belonging to the same group are integrated into one wire. In this manner, the diameter of the catheter 101 can be reduced.

In the second embodiment, at least the acoustic elements 1 belonging to the same group are arranged not to be adjacent to each other. Accordingly, even if the wire is used in common, the detection signals of the acoustic elements 1 do not interfere with each other within the same group. Furthermore, when the number of acoustic elements 1 is defined as N and the number of groups is defined as n, the acoustic elements 1 are arranged such that the acoustic element 1 in the same group is present at every (N/n) -1. In this manner, the incoherence of the detection signal in the same group can be enhanced.

At least in a state where the acoustic elements 1 belonging to the same group are arranged not to be adjacent to each other, the detection signals detected in the group are added, whereby the burden of the wire or the processing can be reduced.

As a method of reducing the number of wires, it can be considered that a channel reduction integrated circuit (IC) that reduces the number of wires by calculating the delay time for each acoustic element 1 is provided. However, when considering the catheter 101 as a disposable device, it is not preferable from the viewpoint of cost.

With respect to this, according to the catheter 101 shown in the second embodiment, it is possible to reduce the number of wires without providing the channel reduction IC.

### [Third Embodiment]

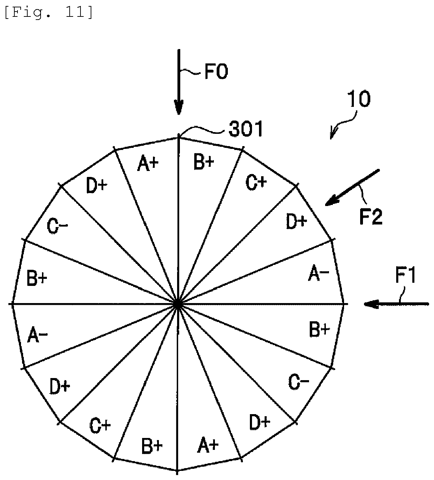

Hereinafter, a third embodiment of the invention will be described with reference to Figs. 11 to 13. In the third embodiment, since the configuration of the photoacoustic catheter system 110 is similar to the configuration illustrated in Fig. 3, and the configuration of the image generation apparatus 102a is similar to the configuration illustrated in Fig. 9, the description here will be omitted. Further, in the description of the third embodiment, Figs. 1 to 3 and 9 are referred to when necessary.

### (Acoustic Element Array)

Fig. 11 is a diagram illustrating a group of acoustic element arrays to be used in a third embodiment.

Fig. 11 is a schematic diagram of the acoustic element array 10 including sixteen acoustic elements 1 as viewed from the axial direction of the catheter 101.

A method of grouping is the same as the method illustrated in Fig. 8, and the sixteen acoustic elements 1 are grouped by the method described in the second embodiment.

However, in Fig. 11, the signal in a part of the acoustic elements 1 is inverted. In Fig. 11, the acoustic elements 1 described as "A-" and "C-" are the acoustic elements 1 in which the signal is inverted. In addition, the acoustic elements 1 described as "A+", "B+", "C+", and "D+" are the acoustic element 1 in which the signal is not inverted. The signal inversion can be easily obtained by the signal processing unit 213 inverting the detection signal from the acoustic element in which the signal is inverted. The signal processing unit 213 specifies whether the detection signal is a detection signal from the acoustic element in which the signal is inverted based on a delay time or the like.

Arrows F0 to F2 will be described below.

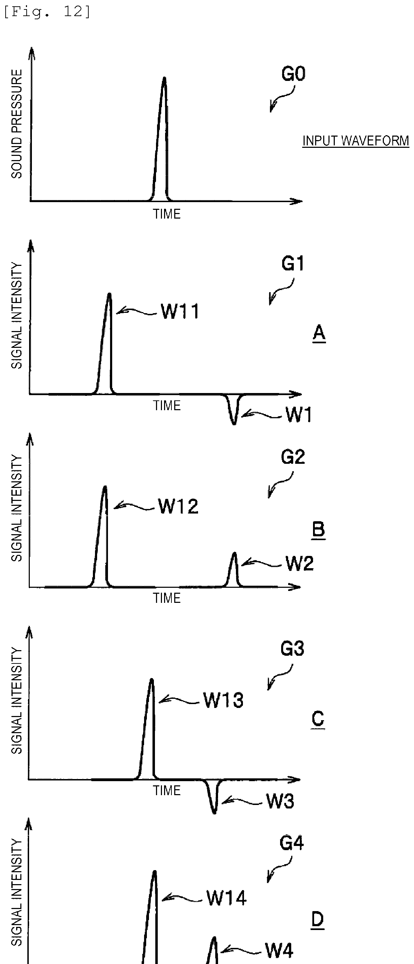

As illustrated in Fig. 11, by disposing the acoustic element 1 for inverting the signal, a noise can be canceled. This matter will be described with reference to Fig. 12.

### (Signal Waveform)

Fig. 12 is a diagram illustrating an input sound wave waveform and a signal waveform in each group.

In each of waveforms illustrated in Fig. 12, a horizontal axis represents time and a vertical axis represents sound pressure (graph G0) or signal intensity (graphs G1 to G4).

As shown in Fig. 11, it is assumed that the acoustic elements 1 are arranged and sound waves as illustrated by the waveform G0 of Fig. 12 are input to the acoustic element array 10 from the direction of the arrow F0.

The waveform G1 in Fig. 12 is a waveform sent from the group of the group "A" to the image generation apparatus 102a. Similarly, a waveform G2 in Fig. 12 is a waveform sent from the group of the group "B" to the image generation apparatus 102a, the waveform G3 is the waveform sent from the group of the group "C" to the image generation apparatus 102a, and the waveform G4 is a waveform sent from the group of the group "D" to the image generation apparatus 102a.

In the graph illustrating the waveform G0, the horizontal axis represents the distance from the sound source, and the vertical axis represents the sound pressure. In the graphs showing the waveforms G1 to G4, the horizontal axis represents the time and the vertical axis represents the sound pressure.

A symbol W1 in the waveform G1 and a symbol W3 in the waveform G3 are detection signals in the acoustic element 1 in which the signal is inverted.

When the signal of a waveform G1 and the signal of a waveform G2 are added, the signal of the symbol W1 in the waveform G1 and the signal of the symbol W2 in the waveform G2 are canceled to each other. The signal of the symbol W11 and the signal of the symbol W12 are strengthened to each other.

Similarly, when the signal of a waveform G3 and the signal of a waveform G4 are added, the signal of the symbol W3 in the waveform G3 and the signal of the symbol W4 in the waveform G4 are canceled to each other. The signal of the symbol W13 and the signal of the symbol W14 are strengthened to each other.

Furthermore, when the Delay & Sum is performed, that is, the signals of the waveforms G1 and G2 are delayed so as to overlap with the signals of the waveforms G3 and G4 and the waveforms G1 to G4 are added, the signals of the symbols W1 to W4 are canceled to each other, and the signals of the symbols W11 to W14 are strengthened.

As described above, the catheter 101 shown in the third embodiment includes, when adding the detection signals detected in each group, a group in which the acoustic element 1 in which the detection signal is inverted is provided so that the detection signal detected after each detection signal detected first in each group is canceled.

In addition, for example, in a case where the sound wave comes flying and coming from the arrow F1 in Fig. 11, the signal processing unit 213 changes the combination of the symbols "+" and "-" so that the direction of a symbol 301 in Fig. 11 becomes the direction of the arrow F1. Such processing can be easily performed by inverting the detection signal from the acoustic elements 1 in which the symbol of the sign is inverted in the signal processing unit 213 without rotating the catheter 101.

In addition, in a case where the sound wave is flying and coming from the direction of the arrow F2, the signal processing unit 213 changes the combination of the symbols "+" and "-" so that the direction of the symbol 301 becomes the direction of the arrow F2, and the arrival time of the detection signal may be delayed.

In this case, it is not necessary to provide a dedicated IC for each of the acoustic elements 1.

### (Flow Chart)

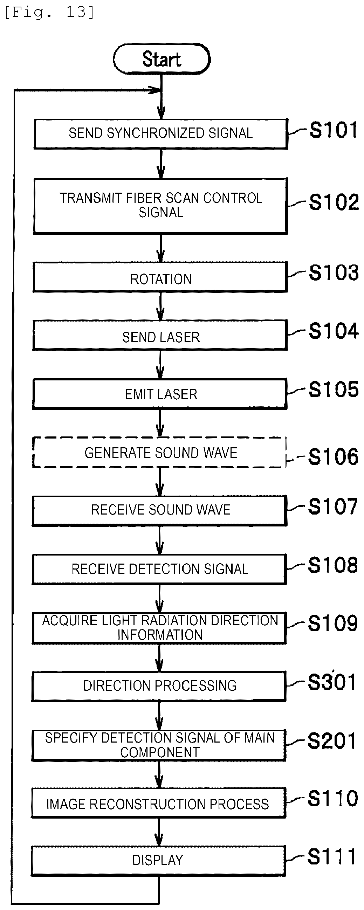

Fig. 13 is a flowchart illustrating a processing procedure in the photoacoustic catheter system to be used in the third embodiment. In Fig. 13, processes similar to those in Fig. 10 are denoted by the same step numbers, and description thereof will be omitted.

In step S109, after the image generation unit 211 of the image generation apparatus 102 acquires the light radiation direction information from the synchronization signal generation device 106 and the scan control device 105, the signal processing unit 213 performs direction processing (S301). The direction processing is to change the combination of "+" and "-" by the signal processing unit 213 so that the light radiation direction (that is, the direction of flight of the sound wave) coincides with the symbol 301 in Fig. 11.

Thereafter, the signal processing unit 213 performs a process of Step S201.

In the above-described second embodiment, since the signals of the symbols W1 and W3 in Fig. 12 are not inverted, when the signals of each group are added, the signals of the symbols W1 and W2, the signals of the symbol W3 and the symbol W4 are strengthened to each other and observed as noise.

According to the third embodiment, since the signal of the symbol W1 of Fig. 2 and the signal of the symbol W2 and the signal of the symbol W3 and the signal of the symbol W4 are canceled, the noise can be canceled.

In this manner, in the third embodiment, when the detection signals detected in each group are added, the acoustic element array 10 is configured so that the detection signal detected after the first detection signal in each group is canceled and the group where the acoustic element 1, in which the detection signal is inverted, is present is provided. In this manner, the noise can be canceled and the detection accuracy of the detection signal of the main component can be improved.

### [Simulation]

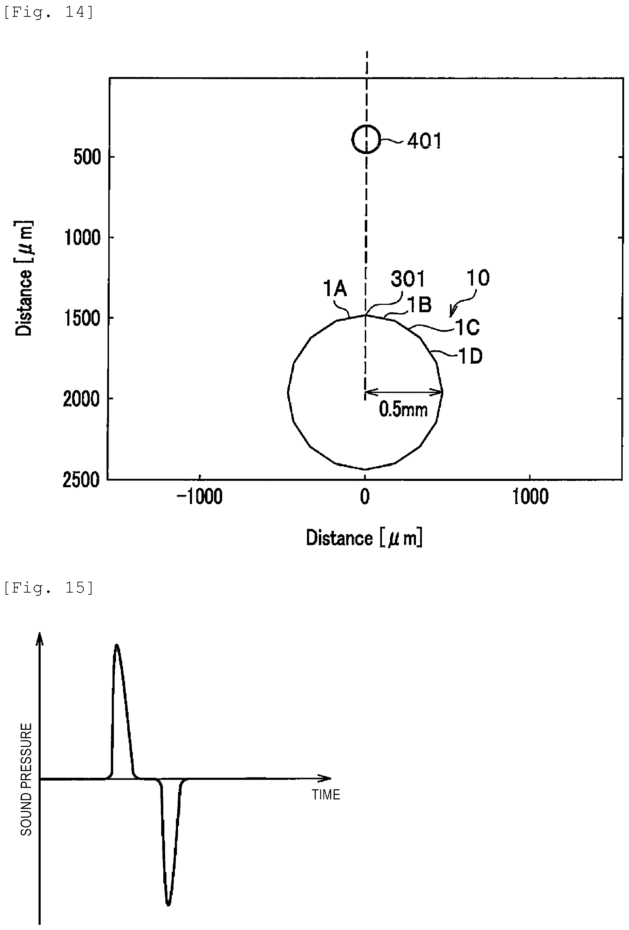

Hereinafter, simulation results of the photoacoustic catheter system 110 shown in the second embodiment and the third embodiment will be shown with reference to Figs. 14 to 19.

Fig. 14 is a diagram illustrating a simulation condition and Fig. 15 is a diagram illustrating a sound wave waveform output from a sound source.

In this simulation, the sound wave having a waveform illustrated in Fig. 15 is output from the sound source indicated by a symbol 401 to the acoustic element array 10 arranged as illustrated in Fig. 14. As illustrated in Fig. 15, the sound wave output here has a form having pulses in a positive direction and a negative direction.

That is, a sound source 401 is arranged on a two-dimensional plane as illustrated in Fig. 14, and the acoustic element array 10 used in the second embodiment and the third embodiment is arranged at the position illustrated in Fig. 14, and a sound wave propagation simulation is performed. It is assumed that both the sound source 401 and the acoustic element array 10 are installed in water simulating a living body. Also, the radius of the acoustic element array 10 is set to 0.5 mm.

The acoustic elements 1A and 1D illustrated in Fig. 14 will be described below.

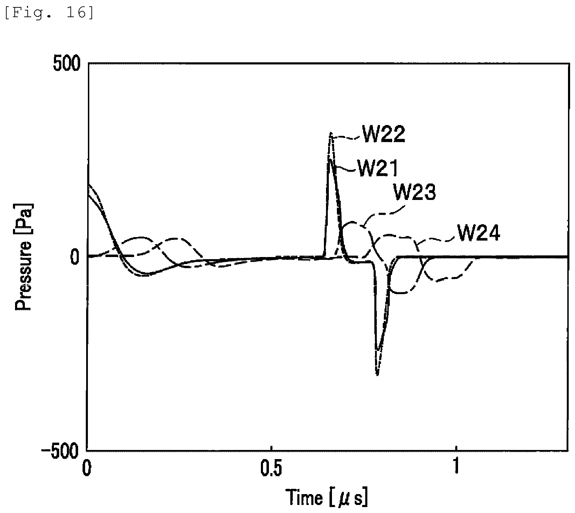

Fig. 16 is a diagram illustrating a waveform of a detection signal in the acoustic elements 1A to 1D among the acoustic elements 1 illustrated in Fig. 14. In Figs. 16 to 19, the horizontal axis represents a time (Time) and the vertical axis represents a sound pressure (pressure). In addition, in Figs. 16 to 19, the waveform detected before 0.5 µs simulates the influence of light radiation.

That is, in Fig. 16, not the detection signal of the group, but the detection signal of each of the acoustic elements 1A to 1D is illustrated.

In Fig. 16, the symbol W21 indicates the detection signal in the acoustic element 1A, the symbol W22 indicates the detection signal in the acoustic element 1B, the symbol W23 indicates the detection signal in the acoustic element 1C, and the symbol W24 indicates the detection signal in the acoustic element 1D, respectively.

As shown in Fig. 16, a waveform having a shape closer to the waveform illustrated in Fig. 15 appears at the earliest time of detection signals (symbols W21 and W22) of the acoustic elements 1A and 1B closest to the sound source.

The detection signal 1C (symbol W23) appears slightly later than the symbols W21 and W22, and the detection signal 1D (symbol W24) appears later than the symbols W21 and W22.

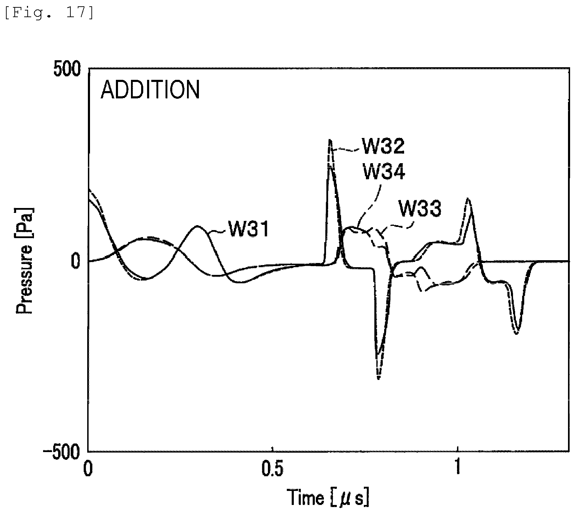

Fig. 17 illustrates a simulation by simulation conditions illustrated in Figs. 14 and 15 and a simulation result of the group shown in the second embodiment.

In Fig. 17, the symbol W31 is the detection signal in the group "A", the symbol W32 is the detection signal in the group "B", the symbol W33 is the detection signal in the group "C", and the symbol W34 is the detection signal in the group "D", respectively. The group here is the group illustrated in Fig. 8, and the sound source 401 (refer to Fig. 14) is arranged in the direction described as the "sound wave" in Fig. 8.

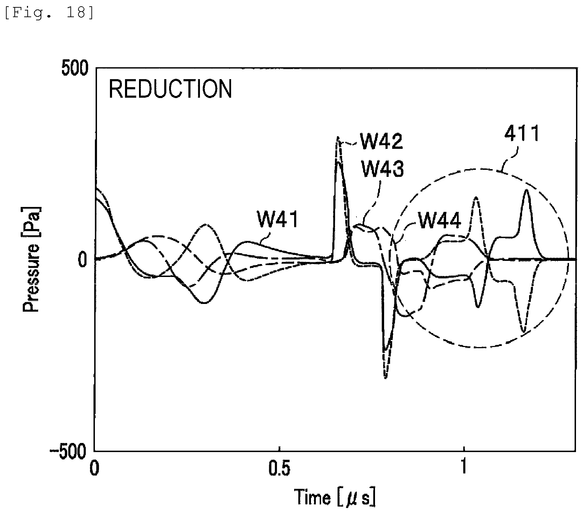

Fig. 18 illustrates a simulation by simulation conditions illustrated in Figs. 14 and 15 and a simulation result of the group shown in the third embodiment. That is, Fig. 18 illustrates the detection signals of each group in a case where the detection signals of a part of the acoustic elements 1 are inverted as illustrated in Fig. 11. In Fig. 18, the symbol W41 is the detection signal in the group "A", the symbol W42 is the detection signal in the group "B", the symbol W43 is the detection signal in the group "C", and the symbol W44 is the detection signal in the group "D", respectively. The group here is the group illustrated in Fig. 11, and the sound source 401 (refer to Fig. 14) is arranged in the direction of the arrow F0 in Fig. 11.

When comparing Figs. 17 and 18, it is found that the signal in a range illustrated in the symbol 411 of Fig. 18 is inverted.

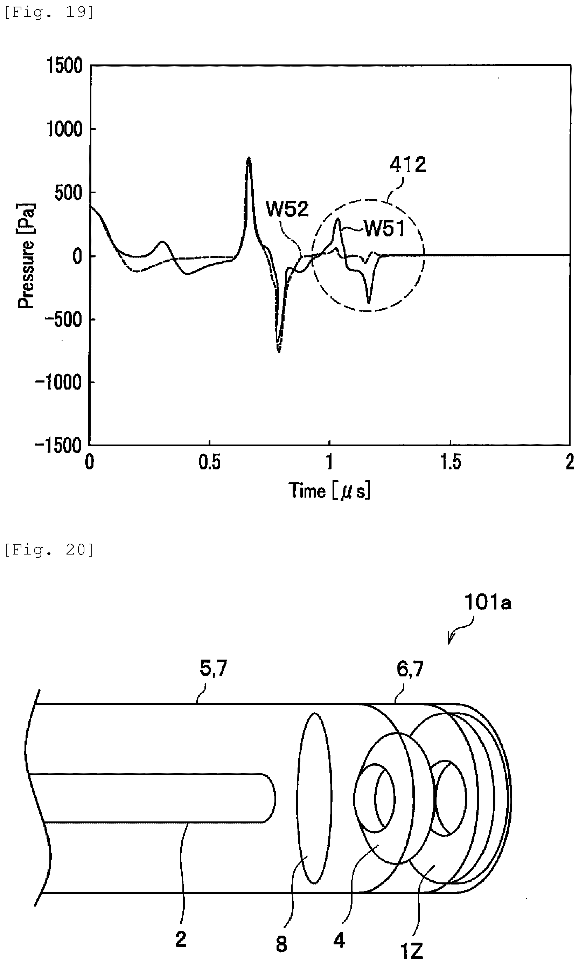

Fig. 19 is a diagram illustrating a result of Delay & Sum in a method used in the second embodiment and the method used in the third embodiment.

In Fig. 19, a symbol W51 indicates a result obtained by performing a Delay & Sum process with respect to the signals W31 to W34 of each group illustrated in Fig. 17. That is, in the second embodiment, the waveform illustrated in the symbol W51 is a signal waveform to be used in an image generating process.

A symbol W52 indicates a result obtained by performing the Delay & Sum process with respect to the signals W41 to W44 of each group illustrated in Fig. 18. That is, in the third embodiment, the waveform illustrated in the symbol W52 is a signal waveform to be used in the image generating process.

When comparing the signal waveform of the symbol W51 and the signal waveform of the symbol W52, in the signal waveform of the symbol W52, it can be found that the noise in the range indicated by a symbol 412 is greatly reduced by inverting the signals as shown by the symbol 411 in Fig. 18.

As shown by the signal waveform of the symbol W51, it is sufficiently possible to extract the main component of the detection signal also by the method of the second embodiment. However, according to the method described in the third embodiment, the noise can be greatly reduced as shown by the symbol W52. Therefore, according to the method described in the third embodiment, the detection accuracy can be greatly improved.

### [Fourth Embodiment]

Fig. 20 is a diagram for illustrating a configuration of a tip end portion of a catheter to be used in a fourth embodiment.

In the catheter 101a illustrated in Fig. 20, a ring-shaped acoustic element 1Z is provided not on the side surface of the catheter 101a as illustrated in Fig. 1 but on the tip end side of the catheter 101a from the reflecting mirror 4. Although the driving device 3 is omitted in Fig. 20, actually the driving device 3 is provided.

Since the configuration is the same as the configuration illustrated in Fig. 1 other than the structure of the acoustic element 1Z, the description here will be omitted.

According to the catheter 101a illustrated in Fig. 20, the sound waves can be detected even in forward scanning.

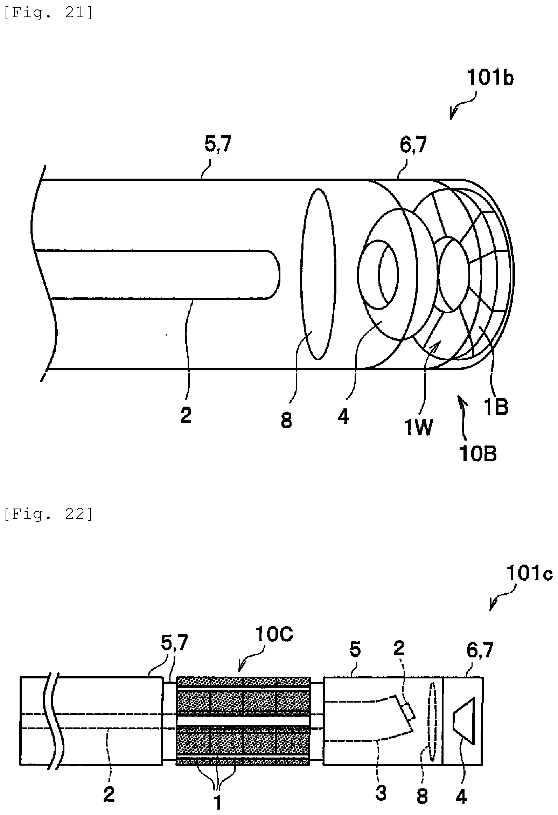

Fig. 21 is a diagram for illustrating another configuration example of the tip end portion of the catheter to be used in the fourth embodiment.

In Fig. 20, one ring-shaped acoustic element 1Z is provided on the tip end side of the catheter 101a from the reflecting mirror 4. However, in a catheter 101b illustrated in Fig. 21, an acoustic array 10B in which a plurality of acoustic elements 1W are arranged in a ring shape is provided on the tip end side of the catheter 101b from the reflecting mirror 4. In this manner, the detection resolution of sound waves when the light is radiated to the front side can be improved.

Although the driving device 3 is omitted in Fig. 21, actually the driving device 3 is provided.

Any one of the catheter 101a to be used in the fourth embodiment and the catheter 101a used in the first to third embodiments may be combined.

### [Fifth Embodiment]

Fig. 22 is a diagram for illustrating a configuration of a tip end portion of a catheter to be used in a fifth embodiment.

A catheter 101c illustrated in Fig. 22 has a plurality of acoustic elements 1 in a side surface circumference direction of the catheter 101c and has an acoustic element array 10C having a plurality of acoustic elements 1 in an axis direction of the catheter 101c. That is, in the acoustic element array 10C illustrated in Fig. 22, the acoustic elements 1 form a two-dimensional array.

In this manner, the detection resolution of sound waves can be improved.

Further, in the present embodiment, the reflecting mirror 4 has the hollow portion Ho so that it is possible to switch between radiation of light to the front and radiation of light to the side. However, the reflecting mirror 4 may not have the hollow portion Ho. In this case, the light can be radiated only to the side.

The invention is not limited to the above-described embodiments and includes various modified examples. For example, the above-described embodiments are described in detail in order to explain the invention for easy understanding, and the invention is not necessarily limited to the structure that includes all, of the described components. Further, a part of a structure in one embodiment can be replaced with a structure in another example, and a structure in one example can also be added to a structure in another embodiment. Moreover, with respect to a part of a structure of each of the embodiments, another structure can be added, deleted, and replaced.

In the first to fourth embodiments, in addition to the above-described PZT element, a PVDF element, a MEMS element, or the like may be used in the driving device 3, or the optical fiber 2 may be rotated by electromagnetic induction.

In addition, a part or all of the individual configurations, the functions, the individual units 210 to 213, the storage device 203, and the like may be designed by integrated circuits and may be realized by hardware. In addition, as illustrated in Figs. 4 and 9, the individual configurations, the functions, and the like may be realized by software by analyzing programs for realizing the functions by a processor such as the CPU 202 and executing the programs by the processor. Information such as the programs, the tables, and the files for realizing the individual functions may be stored in the memory 201 or a recording device such as a solid state drive (SSD) or a recording medium such as an IC card, a secure digital (SD) card, and a digital versatile disc (DVD).

In addition, in the individual embodiments, only control lines or information lines necessary for explanation are illustrated and the control lines or information lines do not mean all control lines or information lines necessary for a product. In actuality, almost all configurations may be connected to each other.

### Reference Signs List

1, 1a to 1f, 1A to 1D, 1W, 1Z acoustic element (acoustic detection unit)
2 optical fiber (optical fiber portion)
3 driving device (driving unit)
4 reflecting mirror (mirror portion)
5, 7 cover
6 transparent cover
8 lens
10, 10B, 10C acoustic element array
101, 101a to 101c catheter (catheter portion)
102, 102a image generation apparatus
103 display device
104 laser generation device (light source unit)
105 scan control device
106 synchronization signal generation device
110 photoacoustic catheter system (catheter system)
210, 210a processing unit
211 image generation unit
212 display processing unit
213 signal processing unit
Ho hollow portion
Rf reflection portion

## Claims

1. A photoacoustic catheter comprising:
a driving unit that drives so as to radiate light in a predetermined direction to an advancing direction of the catheter; and
an acoustic detection unit that receives a sound wave generated by the radiated light,
wherein the acoustic detection unit is provided in a circumference direction of the catheter and provided in a direction having a predetermined angle to an advancing direction of the light.

2. The photoacoustic catheter according to Claim 1,
wherein a plurality of the acoustic detection units are provided in a circumference direction of the catheter.

3. The photoacoustic catheter according to Claim 1,
wherein the acoustic detection unit is provided in a circumference direction of a side surface of the catheter.

4. The photoacoustic catheter according to Claim 1,
wherein a plurality of the acoustic detection units are provided,
wherein a plurality of the acoustic detection units form a group, and
wherein wires of the acoustic detection units belonging to the same group are combined into one wire.

5. The photoacoustic catheter according to Claim 4,
wherein the acoustic detection units belonging to the same group are disposed so that the acoustic detection units are not adjacent.

6. The photoacoustic catheter according to Claim 5,
wherein when the number of acoustic detection units is defined as N and the number of groups is defined as n, the acoustic detection unit of the same group is present at every (N/n)-1.

7. The photoacoustic catheter according to Claim 5,
wherein a signal detected in the group is added.

8. The photoacoustic catheter according to Claim 4,
wherein when the signal detected in each group is added, a group, in which the acoustic detection unit in which the signal is inverted is present, is provided so that the signal detected after a first detected signal in each group is canceled.

9. The photoacoustic catheter according to Claim 1,
wherein the acoustic detection unit in a form of a ring is provided at a tip end of the catheter.

10. The photoacoustic catheter according to Claim 9,
wherein a plurality of acoustic detection units are provided.

11. The photoacoustic catheter according to Claim 1, furt her comprising:
an optical fiber portion that transmits light; and
a mirror portion which is provided in a vicinity of a light emitting end of the optical fiber portion, allows a center portion to transmit light, and has a reflection portion for reflecting the light emitted from the light emitting end of the optical fiber portion to the side portion around the mirror portion,
wherein the driving portion rotates and drives a light emitting end of the optical fiber portion.

12. A photoacoustic catheter system comprising:
a light source unit that emits light;
a catheter having an optical fiber portion for guiding the light emitted from the light source unit;
an image processing unit,
wherein the catheter includes
a driving unit that drives a tip end of the optical fiber portion so as to radiate light in a predetermined direction to an advancing direction of the catheter and
an acoustic detection unit that receives a sound wave generated by the radiated light,
wherein the acoustic detection unit is provided in a circumference direction of the catheter and provided in a direction having a predetermined angle to an advancing direction of the light, and
wherein the image processing unit generates an image based on a signal acquired from the acoustic detection unit.
